# EUROPÄISCHE PATENTSCHRIFT

(11) **EP 1 093 441 B1**
(45) Veröffentlichungstag und Bekanntmachung des Hinweises auf die Patenterteilung: **04.12.2002**
(21) Anmeldenummer: 99926443.5
(22) Anmeldetag: 27.05.1999
(51) Int. Cl.: C02F 1/58, C02F 1/42

(54) **VERFAHREN ZUR RÜCKGEWINNUNG VON FLUORIERTEN ALKANSÄUREN AUS ABWÄSSERN**
METHOD FOR RECOVERING FLUORINATED ALKANOIC ACIDS FROM WASTE WATERS
PROCEDE POUR LA RECUPERATION D'ACIDES ALCANOIQUES FLUORES DANS DES EAUX RESIDUAIRES

(30) Priorität: 02.06.1998 DE 19824615
(43) Veröffentlichungstag der Anmeldung: 25.04.2001
(60) Teilanmeldung: 02075014.7
(73) Patentinhaber: Dyneon GmbH & Co. KG, 84504 Burgkirchen (DE); Celanese Ventures GmbH, 65926 Frankfurt am Main (DE)
(72) Erfinder: FELIX, Bernd, D-84508 Burgkirchen (DE); ZIPPLIES, Tilman, D-84489 Burghausen (DE); FÜHRER, Stephan, D-84508 Burgkirchen (DE); KAISER, Thomas, D-65779 Kelkheim (DE); BUDESHEIM, Armin, D-65207 Wiesbaden- Naurod (DE)
(74) Vertreter: Voortmans, Gilbert J.L.
(86) Internationale Anmeldenummer: EP9903673
(87) Internationale Veröffentlichungsnummer: WO99062830

(56) Entgegenhaltungen:
- EP-A- 0 566 974
- US-A- 4 282 162

## Beschreibung

Für die Polymerisation fluorierter Monomerer in wäßriger Dispersion werden fluorierte Alkansäuren als Emulgatoren eingesetzt, da sie keine telogenen Eigenschaften haben. Vor allem werden die Salze, vorzugsweise die Alkali- oder Ammoniumsalze, von perfluorierten oder teilfluorierten Alkancarbonsäuren oder -sulfonsäuren verwendet. Diese Verbindungen werden durch Elektrofluorierung oder durch die Telomerisation fluorierter Monomerer hergestellt, was mit hohem Aufwand verbunden ist. Es hat deshalb nicht an Versuchen gefehlt, diese Wertstoffe aus Abwässern wiederzugewinnen.

Aus der US-A-5 442 097 ist ein Verfahren zur Rückgewinnung von fluorierten Carbonsäuren in verwertbarer Form aus verunreinigten Ausgangsmaterialien bekannt, wobei man aus diesen Materialien im wäßrigen Medium mit einer hinreichend starken Säure die fluorierte Carbonsäure nötigenfalls freisetzt, diese mit einem geeigneten Alkohol umsetzt und den gebildeten Ester abdestilliert. Als Ausgangsmaterial kann hierbei eine Polymerisationsflotte dienen, insbesondere aus der sogenannten Emulsionspolymerisation, bei der das Fluorpolymer in Form kolloidaler Teilchen mit Hilfe relativ hoher Mengen an Emulgator hergestellt wird. Dieses Verfahren hat sich sehr gut bewährt, setzt aber eine gewisse Konzentration an fluorierter Carbonsäure im Ausgangsmaterial voraus.

Aus der DE-A-20 44 986 ist ein Verfahren zur Gewinnung von Perfluorcarbonsäuren aus verdünnter Lösung bekannt, wobei man die verdünnte Lösung der Perfluorcarbonsäuren in Adsorptionskontakt mit einem schwachbasischen Anionenaustauscherharz bringt und dadurch die in der Lösung enthaltene Perfluorcarbonsäure an das Anionenaustauscherharz adsorbiert, das Anionenaustauscherharz mit einer wäßrigen Ammoniaklösung eluiert und damit die adsobierte Perfluorcarbonsäure in das Elutionsmittel überführt und schließlich die Säure aus dem Eluat gewinnt. Für eine vollständige Elution werden jedoch relativ große Mengen an verdünnter Ammoniaklösung benötigt und außerdem ist dieses Verfahren sehr zeitraubend. Diese Nachteile überwindet das aus der US-A-4 282 162 bekannte Verfahren zur Elution von an basischen Anionenaustauschern adsorbierten fluorierten Emulgatorsäuren, bei dem die Elution der adsorbierten fluorierten Emulgatorsäure aus dem Anionenaustauscher mit einem Gemisch aus verdünnter Mineralsäure und einem organischen Lösungsmittel vorgenommen wird. Bei diesem Verfahren wird durch den Einsatz der Säure gleichzeitig die Regeneration des Austauscherharzes bewirkt.

Es wurde gefunden, daß dieses letztgenannte Verfahren vor allem dann in der betrieblichen Praxis Probleme aufwirft, wenn das verarbeitete Abwasser sehr feinteilige Feststoffe enthält, die oft bisher nicht oder zumindest nicht als störend erkannt worden waren. In diesem Falle setzen sich die das Anionenaustauscherharz enthaltenden Apparaturen mehr oder weniger schnell mit diesen Feststoffen zu, was sich durch erhöhten Strömungswiderstand und verminderte Leistungsfähigkeit bemerkbar macht. Die üblichen vorgeschalteten Filter oder Fritten sind hierbei wirkungslos.

Es wurde außerdem gefunden, daß diese Schwierigkeiten dadurch bedingt sind, daß die feinteiligen Feststoffe durch die Emulgatorsäuren in einer relativ stabilen kolloidalen Feinverteilung gehalten werden. Wenn nun diese Säuren durch das Anionenaustauscherharz aus dem System entfernt werden, wird diese relativ stabile Feinverteilung gestört und der Feststoff fällt aus und verstopft das Austauscherharz. Es wurde somit weiterhin gefunden, daß die Leistungsfähigkeit des aus der US-A-4 282 162 bekannten Verfahrens erheblich gesteigert werden kann und auch für Abwässer geeignet ist, die feinteilige Feststoffe enthalten, wenn man die Verteilung der Feststoffe im Abwasser durch Zugabe eines nichtionischen oder kationischen oberflächenaktiven Zusatzstoffes (Tensid) stabilisiert, bevor man dieses mit dem Anionenaustauscher in Kontakt bringt. Die nichtionischen oder kationischen Tenside werden vom Anionentauscher nicht gebunden.

Die Erfindung bezieht sich somit auf ein Verfahren zur Gewinnung von fluorierten Emulgatorsäuren aus feinverteilte Feststoffe enthaltendem Abwasser, das dadurch gekennzeichnet ist, daß man die im Abwasser feinverteilten Feststoffe mit einem nichtionischen oder kationischen Tensid beziehungsweise einer analog wirkenden oberflächenaktiven Substanz stabilisiert und anschließend die fluorierten Emulgatorsäuren an ein Anionentauscherharz bindet und aus diesem die fluorierten Emulgatorsäuren eluiert.

Als Abwässer kommen Prozeßabwässer in Betracht, in denen oberflächenaktive fluorierte Alkansäuren enthalten sind. Besonders geeignet ist das Verfahren für Abwässer aus der Polymerisation fluorierter Monomerer nach dem sogenannten Emulsionsverfahren, bei dem das fluorierte Monomere unter mildem Rühren unter Einsatz einer relativ hohen Konzentration an fluorierter Emulgatorsäure in ein feinteiliges Polymeres überführt wird, das in feindisperser, kolloidaler Form vorliegt und wobei der so gewonnene Latex nach Erreichen der gewünschten Feststoffkonzentration, beispielsweise durch intensives Rühren, koaguliert wird, wodurch sich das Polymer als feines Pulver abscheidet.

Es wurde gefunden, daß bei der bekannten Aufarbeitung vor allem relativ niedermolekulare Polymeranteile Schwierigkeiten machen, wobei sich diese niedermolekularen Polymeren besonders störend bemerkbar machen, wenn das Polymerisationsverfahren zu einer breiten Molgewichtsverteilung führt. Auch bei solchen "schwierigen" Abwässern zeigt sich die Leistungsfähigkeit des erfindungsgemäßen Verfahrens.

Es wurde weiterhin gefunden, Feststoffe abzutrennen, bevor das Abwasser mit dem Ionenaustauscherharz in Kontakt gebracht wird (deutsche Patentanmeldung 198 24 614.5 vom 02.06.1998 mit dem Titel "Verfahren zur Rückgewinnung von fluorierten Alkansäuren aus Abwässern"). Nachteilig ist hierbei jedoch der große apparative Aufwand für die Feststoffabtrennung sowie die Menge der zuzugebenden Hilfschemikalien (zum Beispiel Kalkmilch, Aluminiumsalze, Flockungshilfsmittel). Insbesondere bei geringen Feststoffkonzentrationen sind für eine vollständige Entfernung der Kolloidalen größere Mengen der Chemikalien erforderlich, die nur begrenzt in der Feststoffabtrennung wieder entfernt werden.

Bei dem erfindungsgemäßen Verfahren wird der apparative und chemische Aufwand erheblich reduziert, da bereits die Zugabe geringer Mengen eines vorzugsweise gut biologisch abbaubaren Tensids für eine Stabilisierung der Kolloide genügt und einen störungsfreien Betrieb des Ionenaustauschers gewährleistet.

Die Adsorption der Emulgatorsäuren an Austauscherharze kann in an sich bekannter Weise erfolgen. Geeignet sind insbesondere stark basische Anionenaustauscherharze, wie sie beispielsweise unter den Handelsbezeichnungen ®AMBERLITE IRA-402, ®AMBERJET 4200 (beide: Rohm & Haas), ®PUROLITE A845 (Purolite GmbH) oder ®LEWATIT MP-500 (Bayer AG) erhältlich sind.

Die Adsorption kann in an sich bekannter Weise erfolgen, wobei die Austauscherharze in den üblichen Apparaturen wie Röhren oder Säulen angeordnet sind, die von dem Abwasser durchströmt werden.

Die Elution der gebundenen Emulgatorsäuren erfolgt ebenfalls in an sich bekannter Weise, wobei das Verfahren nach der US-A-4 282 162 bevorzugt wird.

Für die Gewinnung der Emulgatorsäuren in der erforderlichen hohen Reinheit für den Einsatz in der Polymerisation eignen sich beispielsweise die Verfahren nach der obengenannten US-A-5 442 097 oder das Verfahren nach der US-A-5 312 935, bei dem man das Eluat zunächst weitgehend wasserfrei macht und dann mit Oxidationsmitteln behandelt.

Das nach der Adsorption der Emulgatorsäuren verbleibende Abwasser wird je nach Gehalt an sonstigen Stoffen in bekannter Weise aufgearbeitet.

Die Erfindung wird in den folgenden Beispielen noch näher erläutert.

Beispiele 1 bis 4 und Vergleichsbeispiel

Als Ausgangsmaterial dient ein Abwasser aus der Copolymerisation von Tetrafluorethylen und Perfluor(n-propyl-vinyl)ether, bei der das Ammoniumsalz der n- und iso-Perfluoroctansäure (PFOS) als Emulgator eingesetzt wird. Die PFOS-Konzentration beträgt 750 mg/l.

In einem Rührgefäß werden 1000 g dieser Flotte mit 0,1 g des nichtionischen Tensids ®TRITON X-100 (Rohm & Haas, p-Octylphenol-oxethylat, CAS-Nr. 9002-93-1) beziehungsweise ®GENAPOL UD 088 (Hoechst AG, Fettalkoholpolyglykolether) versetzt und gerührt.

Etwa 50 ml eines handelsüblichen stark basischen Ionenaustauscherharzes (®AMBERLITE IRA-402, Rohm & Haas; Styrol-Divinylbenzol-Typ, Anion: Chlorid, Gel, Totalkapazität 1,3 eq/l, Schüttgewicht 710 g/l) werden in eine mit Glasfritte versehene zylindrische Glassäule (Länge 25 cm, Durchmesser 16 mm) gegeben und mit Wasser gespült. Zur Beladung des Ionentauschers wird die Lösung mit einer Pumpe im Aufstrom durch das Bett gefördert. Das austretende Wasser wird in mehreren Proben gesammelt und die PFOS-Konzentration bestimmt. Der Druckverlust über das Ionenaustauscherbett wird mit einem Manometer gemessen. Das austretende Wasser wird in mehreren Proben gesammelt und die PFOS-Konzentration bestimmt.

Der Beladungsversuch ohne Tensidzugabe (Vergleichsbeispiel) mußte abgebrochen werden, da infolge von ausgefallenem Polymer ein Druckverlust von über 1 bar/m überschritten wurde und das Harz deutlich verklebte.

| Menge Abwasser [ml] | | Beispiel 1 | Beispiel 2 | Beispiel 3 | Beispiel 4 | Vergleichsbeispiel |
|---|---|---|---|---|---|---|
| | Tensid: | ®GENAPOL | ®TRITON | ®TRITON | ®TRITON | ohne |
| | Konzentration: | 100 mg/l | 100 mg/l | 200 mg/l | 400 mg/l | --- |
| | | Druckverlust über lonentauscherbett [bar/m] | | | | |
| 0 | | 0,12 | 0,24 | 0,24 | 0,16 | 0,20 |
| 200 | | 0,16 | 0,20 | 0,24 | 0,16 | 0,40 |
| 300 | | 0,12 | 0,24 | 0,24 | 0,24 | |
| 400 | | 0,12 | 0,24 | 0,24 | | 1,60 (Abbruch) |
| 500 | | 0,12 | 0,24 | | 0,24 | |
| 600 | | 0,12 | 0,24 | | 0,24 | |
| 1000 | | 0,12 | | | | |
| PFOS im Abwasser [mg/l] | | 2,5 | 5,1 | 4,2 | 4,2 | 3,3 |

### Beispiel 5

Aus Methanol, konzentrierter Schwefelsäure (96 %) und Wasser werden 150 ml einer Elutionslösung gemischt (Massenanteile 89 %, 7 %, 4 %). Die Ionentauschersäule wird nach der Beladung zunächst mit 100 ml Wasser gespült, um Reste des Abwassers aus der Säule zu entfernen. Dann wird die Elutionslösung mit einer Lineargeschwindigkeit von 0,5 m/h über die Säule gefördert und aufgefangen. Die Säule wird abschließend mit weiteren 50 ml Waser gespült. Die Elutionslösung enthält etwa 95 % der im Abwasser eingesetzten Emulgatorlösung.

## Patentansprüche

1. Verfahren zur Gewinnung von fluorierten Emulgatorsäuren aus feinverteilte Feststoffe enthaltendem Abwasser, **dadurch gekennzeichnet, daß** man die im Abwasser feinverteilten Feststoffe mit einem Tensid beziehungsweise einer oberflächenaktiven Substanz stabilisiert und anschließend die fluorierten Emulgatorsäuren an ein Anionentauscherharz bindet und aus diesem die fluorierten Emulgatorsäuren eluiert.

2. Verfahren nach Anspruch 1, **dadurch gekennzeichnet, daß** ein Abwasser aus der Polymerisation fluorierter Monomerer eingesetzt wird.

3. Verfahren nach Anspruch 1 oder 2, **dadurch gekennzeichnet, daß** die in Feststoffe überführbaren Anteile ausgefällt werden.

4. Verfahren nach einem oder mehreren der vorhergehenden Ansprüche, **dadurch gekennzeichnet, daß** das eingesetzte Anionenaustauscherharz stark basisch ist.

5. Verfahren nach einem oder mehreren der vorhergehenden Ansprüche, **dadurch gekennzeichnet, daß** die Elution mit einem Gemisch aus verdünnter Mineralsäure und einem organischen Lösungsmittel erfolgt.

## Claims

1. Process for the recovery of fluorinated emulsifier acids from wastewater which contains finely dispersed solids, **characterized in that** the solids which are finely dispersed in the wastewater are stabilized using a surfactant or a surface-active substance and the fluorinated emulsifier acids are subsequently bound on an anion-exchange resin and the fluorinated emulsifier acids are eluted from the latter.

2. Process according to Claim 1, **characterized in that** wastewater from the polymerization of fluorinated monomers is used.

3. Process according to Claim. 1 or 2, **characterized in that** the material which is convertible into solids is precipitated.

4. Process according to one or more of the preceding claims, **characterized in that** the anion-exchange resin used is a strong base anion-exchange resin.

5. Process according to one or more of the preceding claims, **characterized in that** elution is carried out using a mixture of dilute mineral acid and an organic solvent.

## Revendications

1. Procédé d'extraction d'acides émulsifiants fluorés à partir d'eaux résiduaires, **caractérisé en ce que** l'on stabilise les matières solides finement divisées dans l'eau résiduaire à l'aide d'un agent tensioactif ou d'une substance à activité superficielle et **en ce que** l'on procède ensuite à la fixation des acides émulsifiants fluorés sur une résine échangeuse d'anions et à l'élution hors de celle-ci des acides émulsifiants fluorés.

2. Procédé selon la revendication 1, **caractérisé en ce que** l'on utilise une eau résiduaire provenant de la polymérisation de monomères fluorés.

3. Procédé selon la revendication 1 ou 2, **caractérisé en ce que** les portions que l'on peut convertir en matières solides sont précipitées.

4. Procédé selon l'une quelconque des revendications précédentes, **caractérisé en ce que** la résine échangeuse d'anions est fortement basique.

5. Procédé selon l'une quelconque des revendications précédentes, **caractérisé en ce que** l'élution se fait à l'aide d'un mélange obtenu à partir d'un acide minéral dilué et d'un solvant organique.
